# EUROPEAN PATENT SPECIFICATION

(11) **EP 3 216 416 B1**
(45) Date of publication and mention of the grant of the patent: **09.10.2019**
(21) Application number: 17163161.7
(22) Date of filing: 07.04.2014
(51) Int. Cl.: A61B 90/00, A61B 90/50

(54) **REFERENCE DEVICE FOR SURGICAL NAVIGATION SYSTEM**
REFERENZVORRICHTUNG FÜR CHIRURGISCHES NAVIGATIONSSYSTEM
DISPOSITIF DE RÉFÉRENCE POUR SYSTÈME DE NAVIGATION CHIRURGICALE

(30) Priority: 04.04.2014 US 201414245170
(43) Date of publication of application: 13.09.2017
(62) Divisional of application: 14887779.8
(73) Proprietor: IZI Medical Products, LLC, Owings Mills, Maryland 21117 (US)
(72) Inventor: ROSSNER, Holger-Claus, Owings Mills, MD Maryland 21117 (US)
(74) Representative: Higgs, Jonathan

(56) References cited:
- WO-A1-2005/023128
- WO-A1-2012/110082
- WO-A2-01/76499
- WO-A2-2004/014219
- WO-A2-2004/069073
- WO-A2-2013/112623
- US-A1- 2009 306 499
- US-B1- 6 860 877

## Description

The present invention relates generally to surgical navigation systems. More particularly, the present invention relates to a referencing device for a surgical navigation system and a marker carrier unit for use in a referencing device.

Surgical navigation systems are employed in a variety of surgical applications, for example, in neurosurgery, oral, maxillofacial and facial surgery, ear nose and throat (ENT) surgery or also for limb implantation in orthopedic surgery. Based on three-dimensional patient image data, which are obtained by means of X-ray images, computer tomography (CT), magnetic resonance tomography (MRT) and/or positron emission tomography (PET), surgical navigation systems of this type enable the position of medical instruments to be visualized in real-time in the patient image data in order to thereby assist the surgeon during operable procedures.

To this end, it may be necessary to record and monitor the position and orientation of the patient or a specific body part on which a surgical procedure is to be carried out - also referred to as "tracking." Conventional referencing devices have been used usually comprising reference frames to which marking elements such as light-reflecting, spherical marker elements are attached. The light-reflecting spherical marker elements allow a stereo camera system of the navigation system to record the precise position and orientation of the referencing device.

Medical devices with adjustable positions are known from WO2013/112623, WO2004/069073, WO2005/023128, WO01/76499, WO2004/014219, US6860877 and WO2012/110082.

Conventional navigation systems and/or referencing devices are known, for example, from documents DE 10 2011 054 730 A1, DE 698 33 881 T2, DE 10 2010 060 914 A1 or DE 60 2004 004 158 T2. WO 2006/012491 discloses marker elements together with a unit carrying the marker elements - referred to as reference frames - as a single disposable unit which can be produced by injection molding. However, traditional navigation systems do not always allow for the desired positioning and orientation of the referencing device, for example, due to structural limitations in the design of its arranged configuration and/or restrictions in movement such as limited multiple ranges of motion and/or operating degrees of freedom.

Another concern may include operating and maintaining a sterile environment during surgical procedures. Medical devices, such as referencing devices must also be sterile. Within such an environment, marker elements may be removably attached, for example, by means of a standardized clip attachment to pins arranged on the referencing device. The referencing device may thus be sterilized without marker elements and new, sterile, disposable marker elements may be utilized for each use. Conventional corresponding marker elements are known, for example, from document DE 10 2009 019 986 A1.

In order to deduce the position and orientation of a patient (or as the case may be, the body part of a patient on which a surgical procedure is to take place), and in order to produce a correct reference to the 3D image data, it is necessary to calibrate the surgical navigation system by executing a registration step. Various reference points are thereby successively localized on the patient using a navigation apparatus and correlated with corresponding points in the 3D image data.

The registration process determines the geometric relationship between the anatomic structures of interest and the 3-dimensional (3D) computer image constructed, for example, from the preoperative CT scan. Registration involves two steps. First, the reference sensor is secured to a non-mobile structure. Then, a registration tip, for example, is used sequentially to touch pre-selected registration points (e.g., fiducial markers). Registration points may be any anatomic structures that are recognizable on the preoperative image (e.g. teeth, skin, bone). Each time a registration point is touched with the registration tip, the computer records the location of the position sensor and the reference sensor. Using, for example, at least three registration points, the computer calculates the physical position of the anatomic structure with respect to the reference sensors. The computer then uses this registration information to measure the position of the pencil relative to the preoperative CT scan. The patient's body part can be mobilized freely without the need to re-initialize the registration process, because the reference sensor is rigidly attached to the relevant structure of the patient. By way of this registration, a correct, spatial reference between the 3D image data and the position and orientation of the body part of the patient can be produced.

In particular, in the case of surgical procedures involving the brain, it is usually not possible to simply be limited to reference points in the operating area for a necessarily precise registration, but rather it is necessary, in the vast majority of cases, to select a plurality of reference points at different locations on the body of the patient. Since for this purpose unhindered access to these locations on the body of the patient is necessary, registration must thus take place before the patient can be finally prepared for the actual surgical procedure and covered in a sterile manner in the areas outside of the operating area.

As a practical matter, and as it pertains to the registration device itself, following a successful registration procedure necessarily means he registration device must be considered as being potentially contaminated. Thus, appropriate measures for protecting the patient must be taken before the image-guided surgical procedure using the navigation system can take place. As such, the reference frame is thus usually detached from the fixation unit, sterilized, and provided with new sterile marker elements and reconnected to the fixation unit. The fixation unit as well as the interface between the fixation unit and the reference frame must next be draped and/or otherwise covered. To achieve this, holes are typically generated in medical drapes in order to allow the reference frame or its components to protrude therethrough and to subsequently attach to the fixation unit. Additional care to secure and maintain medical drapes is also provided in order to achieve a covering considered at least sufficiently secure. From a user perspective, this approach is presented as less than desirable since, on the one hand, the effort is labor intensive and significant staff effort is required in order to provide the necessary draping and covering for operational procedure. And, on the other hand, the draping and covering is often regarding as insufficiently secure for operating procedures. This risks the sterility of the operating environment and loss of time in addressing the same.

It is, therefore, an object of the present invention to overcome the deficiencies of the prior art to provide an improved apparatus capable of providing increased range of motion in at least multiple to an infinite amount of directions while more easily achieving and maintaining a sterile operating environment. It is a further goal of the present invention to provide a method and apparatus that achieves and maintains a dependable fixed position of the referencing device during operational procedures that eliminates the need to recalibrate the system.

### SUMMARY

The invention resides in a device according to claim 1.

As such, those skilled in the art will appreciate that the concept upon which this disclosure is based may readily be utilized as a basis for the designing of other structures, methods and systems for carrying out the several purposes of the present invention.

Still other aspects, features and advantages of the present invention are readily apparent from the following detailed description, simply by illustrating a number of exemplary examples and implementations, including the best mode contemplated for carrying out the present invention. Accordingly, the drawings and descriptions are to be regarded as illustrative in nature, and not as restrictive.

### BRIEF DESCRIPTION OF THE DRAWINGS

The accompanying drawings, which are incorporated herein and constitute part of this specification, illustrate exemplary examples of the invention, and, together with the general description given above and the detailed description given below, serve to explain the features of the invention. The invention is clearly illustrated in figures 6, 7, 12 and 13 whereas figures 8 and 9 illustrate an exemplary design not forming part of the invention.

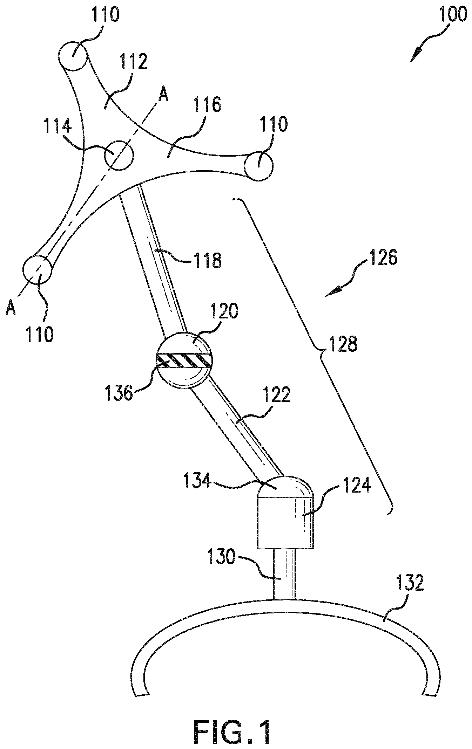

FIG. 1 is a perspective view of a referencing device for a surgical navigation system according to an example.

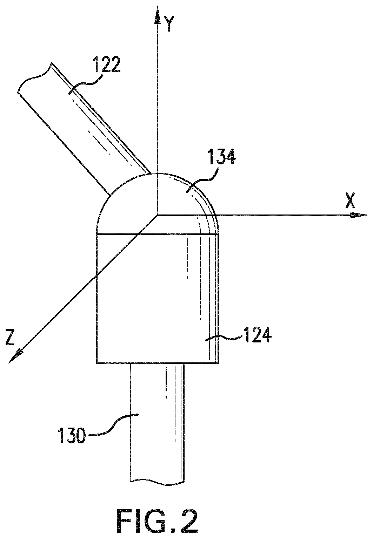

FIG. 2 is an exploded view of an exemplary ball and socket joint disposed near one end of an articulated arm of the referencing device according to one example.

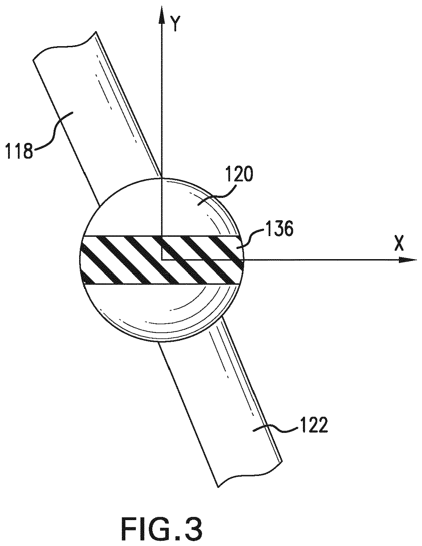

FIG. 3 is an exploded view of an exemplary rotary joint disposed along an articulated arm of the referencing device according to one example.

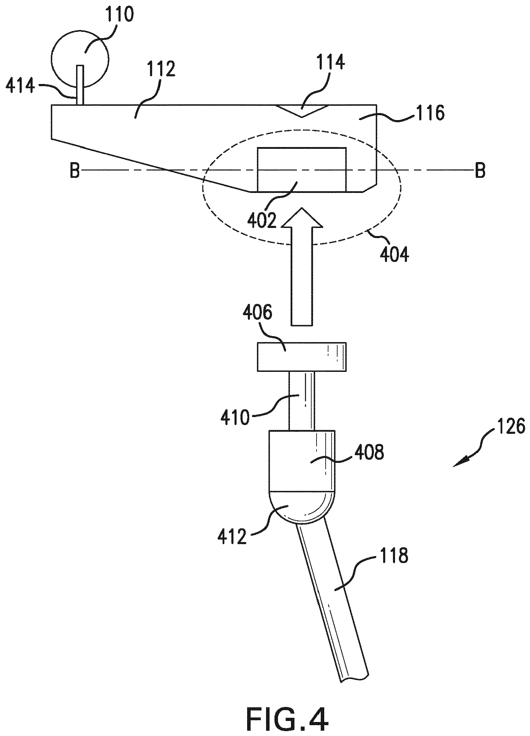

FIG. 4 is side view depicting the assembly of an attachment foot in connection with an articulated arm being mated with a marker carrier unit according to one example of the present invention.

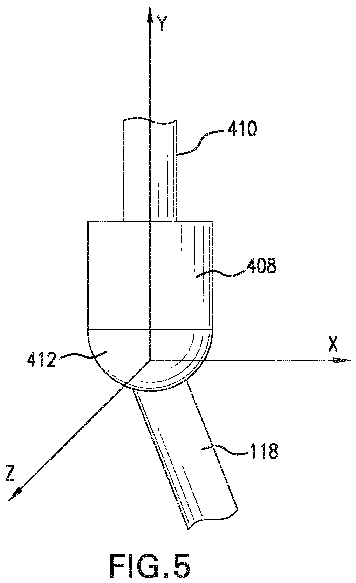

FIG. 5 is an exploded view of another exemplary ball and socket joint disposed near another end of an articulated arm of the referencing device according to one example.

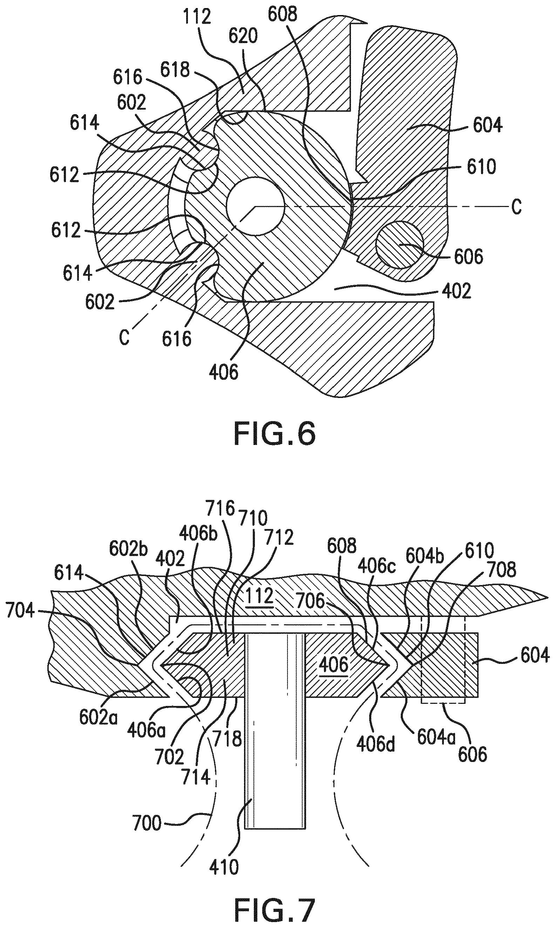

FIG. 6 is a top view of an exemplary design of an attachment foot mated in an exemplary recess of a marker carrier body according to the present invention.
FIG. 7 is a cross sectional view taken along C - C of FIG. 6 according to the present invention.

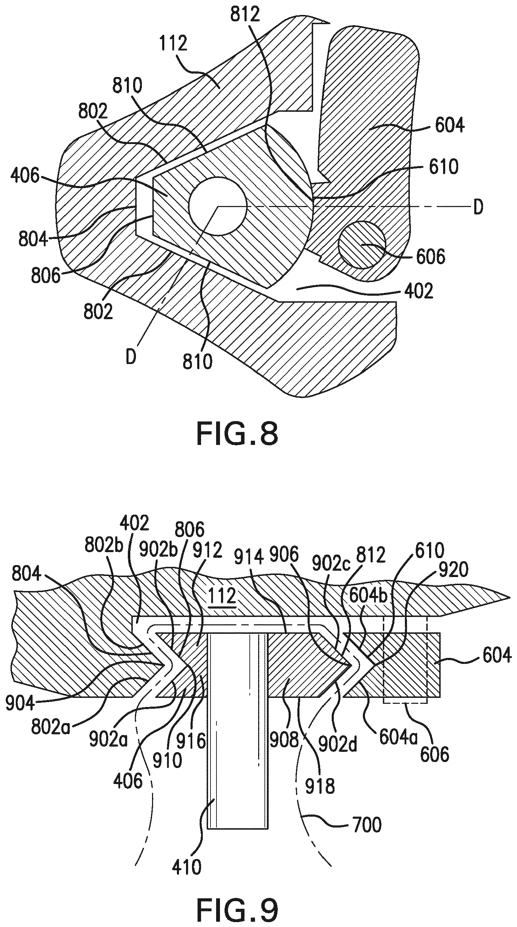

FIG. 8 is a top view of an exemplary design of an attachment foot mated in an exemplary recess of a marker carrier body according to one example.
FIG. 9 is a cross sectional view taken along D - D of FIG. 8 according to one example.

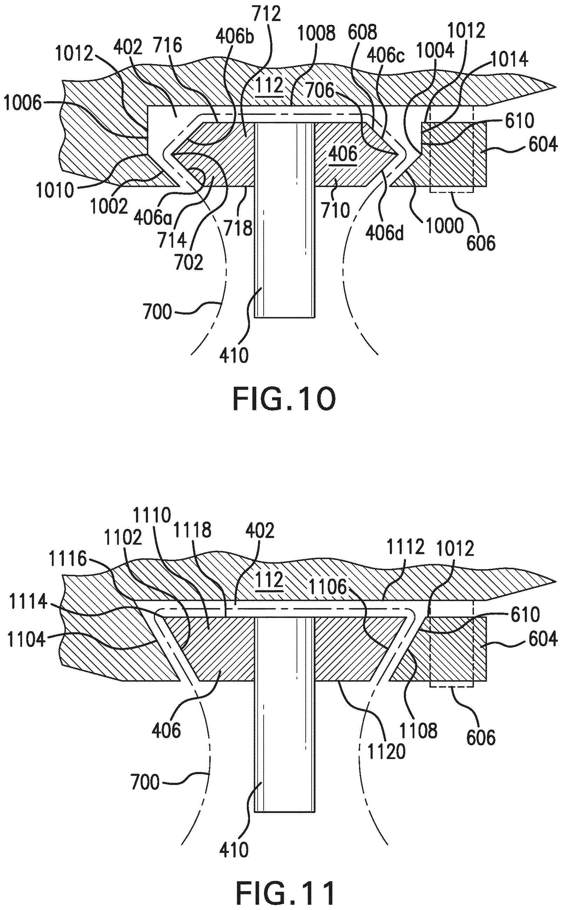

FIG. 10 is another side view of an assembly of an exemplary attachment foot being secured to an exemplary marker carrier body via an exemplary design of a clamp lever according to one example.
FIG. 11 is another side view of an assembly of an exemplary attachment foot being secured to an exemplary marker carrier body via an exemplary design of a clamp lever according to one example.

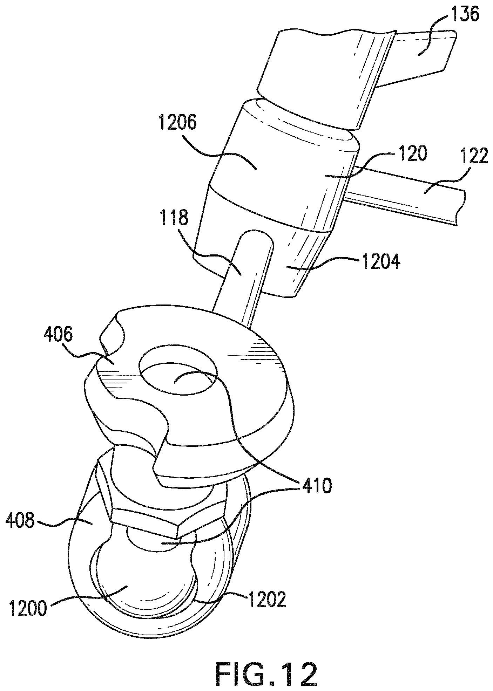

FIG. 12 is a partial view of an exemplary attachment foot attached to an articulated arm via a ball and socket joint according to the present invention.

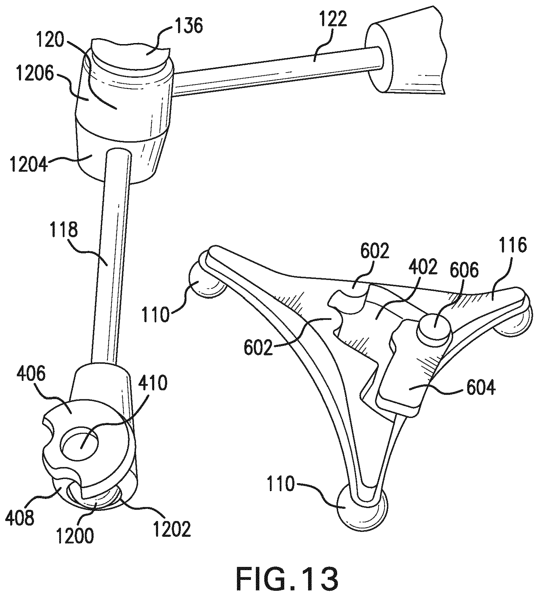

FIG. 13 illustrates an example of a marker carrier unit for assembly with the illustrated exemplary attachment foot according to the present invention.

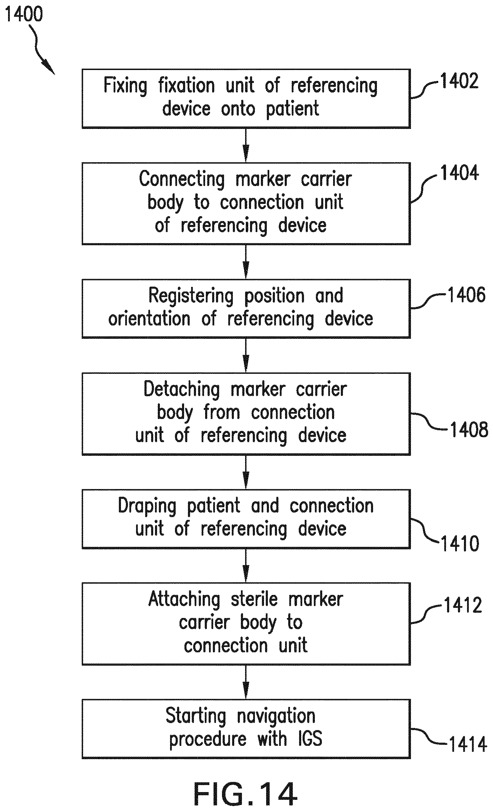

FIG. 14 illustrates steps for preparing and affixing the disclosed referencing device onto a patient for a surgical navigation procedure according to an example of the present invention.

### DETAILED DESCRIPTION OF THE PREFERRED EXAMPLES

### Definitions

Where the definition of terms departs from the commonly used meaning of the term, applicant intends to utilize the definitions provided below, unless specifically indicated.

For the purposes of the present invention, directional terms such as "top", "bottom", "upper", "lower", "above", "below", "left", "right", "horizontal", "vertical", "upward", "downward", etc., are merely used for convenience in describing the various examples of the present invention.

For purposes of the present invention, the term "ball and socket joint" (also referred to a ball joint) refers to a joint, as in a mechanical device, that permits rotary movement in all directions through the movement of a ball in a socket. The ball and socket joint is a joint in which the ball-shaped surface of one rounded member fits into a cup-like depression of another member. The distal member is capable of motion around an indefinite number of axes, which have one common center. It enables the member to move in many planes (almost all directions).

For purposes of the present invention, the term "distal" refers being situated away from a point of attachment or origin or a central point.

For purposes of the present invention, the term "drape" refers to the sterilized cloths that mark off an operative field. Typically the aforementioned cloths are arranged over a patient's body during an examination or treatment or during surgery and are designed to provide a sterile field around the area. "Draping" refers to the process thereof.

For purposes of the present invention, the term "Image-guided surgery" (IGS) refers to surgical procedures where the surgeon employs tracked surgical instruments in conjunction with preoperative or intraoperative images in order to indirectly guide the procedure. Image-guided surgery is part of the wider field of computer-assisted surgery. During a surgical procedure, the IGS tracks the probe position and displays the anatomy beneath it as, for example, three orthogonal image slices on a workstation-based 3D imaging system. Existing IGS systems use different tracking techniques including mechanical, optical, ultrasonic, and electromagnetic.

For purposes of the present invention, the term "indicia" refers distinctive marks, characteristic markers or indications.

For purposes of the present invention, the term "proximal" refers to being next to or nearest the point of attachment or origin, a central point, or the point of view; especially located toward the center of the body - compare distal. For purposes of the present invention, the term "distal" refers to the direction opposite the "proximal" direction.

For purposes of the present invention, the term "registering" refers to a process for determining the geometric relationship between an anatomic structure(s) of interest and a 3-dimensional (3D) computer image constructed, for example, from the preoperative CT scan. By way of this registration, a correct, spatial reference between the 3D image data and the position and orientation of the body part of the patient, observed by means of referencing device, can be produced.

For purposes of the present invention, the term "rotary joint" refers to a freely moving joint in which movement is limited to rotation; the rotary joint may be considered as a flexible joint that connects a stationary object with a rotating object in a piece of machinery, for example, factory and medical equipment.

For purposes of the present invention, the term "surgical navigation" refers to computer assisted surgery (CAS) representing a surgical concept and set of methods that use computer technology for pre-surgical planning and for guiding or performing surgical interventions. CAS is also known as computer aided surgery, computer assisted intervention, image guided surgery and surgical navigation.

For purposes of the present invention, the term "surgical navigation system" refers a system that allows visualization of an operative site and surgical instruments simultaneously and relates them to the patient's diagnostic images (e.g., computed tomographic (CT) scans and magnetic resonance imaging (MRI)). A surgical navigation system is used to guide the surgeon's movements during an operation. It may display the real-time position of each instrument and anatomical structure. These systems are used in orthopedics, ENT, neurology and other surgical specialties. Real-time observations occur via MRI, scanner, video camera or another imaging process. Navigation data are incorporated into the image to help the surgeon determine precise position within the organism. Medical imaging is sometimes used to plan an operation before surgery. Data integration enables the system to compare the actual position of the target object with the ideal location established during the planning phase. Such systems may be mechanical, electromagnetic or optical. The most common are optical devices, either passive or active. In the former, cameras locate specific markers such as reflective targets, particular shapes or colors. Active systems locate LEDs.

For purposes of the present invention, the term "x-direction" refers to the direction aligned with the x-axis of a coordinate system.

For purposes of the present invention, the term "y-direction" refers to the direction aligned with the y-axis of a coordinate system.

For purposes of the present invention, the term "z-direction" refers to the direction aligned with the z-axis of a coordinate system.

### Description

The invention will now be described with reference to the drawing figures, in which like reference numerals refer to like parts throughout. The following detailed description is of example examples of the presently claimed invention with references to the accompanying drawings. Such description is intended to be illustrative and not limiting with respect to the scope of the present invention. Such examples are described in sufficient detail to enable one of ordinary skill in the art to practice the subject invention, and it will be understood that other examples may be practiced with some variations without departing from the scope of the subject invention.

Turning to FIG. 1, a referencing device 100 is illustrated for positioning and mounting one or more marker elements 110 disposed on a marker carrier body 112 of a marker carrier unit 116. In the disclosed example, marker carrier body 112 is substantially designed in as a three-armed unit wherein in the region of the end of each arm a marker element 110 is respectively arranged. Marker elements 110 may be designed as spherical marker elements including retro-reflective marker spheres, also referred to as passive reflective markers, and are widely used in image guidance systems. Examples of retro-reflective marker spheres may include those used to aid registration and instrument tracking during image guided surgery procedures such as neurological procedures, spine procedures and orthopedic procedures. Examples may include retro-reflective marker spheres having a high coefficient of retro-reflection on the external surface to provide feedback to the system/camera. Such surfaces may consist of micro glass spheres that reflect light. Depending on the medical application, different numbers and arrangements of retro-reflective marker spheres may be mounted on various types of surgical tools that may be used including that disclosed herein. Once mounted on a surgical probe, retro-reflective marker spheres provide an accuracy reference point for the surgical probe in three-dimensional space.

Disclosed examples provide the attachment of maker carrier unit 116 to a connection unit 126, for example, at a prescribed location 114 of marker carrier body 112. Prescribed location 114 may be located anywhere at the marker carrier unit 116. In one exemplary configuration, connection unit 126 includes an articulated arm 128 comprising a plurality of arm extensions 118, 122 and joints 408 (FIG. 4), 120 and 124. While two arm extensions 118, 122 and three joints 408, 120 and 124 are described and illustrated in the exemplary drawings, it is readily appreciated that more arm extensions and joints may be employed by the disclosed invention, for example, to facility increased mobility and degrees of freedom in motion of articulated arm 128 and, hence, referencing device 100. Connection unit 126 may also comprise an attachment unit 132 and attachment foot 406 (FIG. 4), as described below.

Arm extension 118 connects to marker carrier body 112 of marker carrier unit 116 at a first end and is configured to join with joint 120 at a second end. Arm extension 118 is a rigid arm extension and may be composed of stainless steel, medical grade steel, materials. In the disclosed example, joint 120 may include a rotary joint for rotating arm extension 118 in the x-direction and y-direction direction (e.g., see FIG. 3). Turning to FIGS. 12 and 13, the exemplary configuration of joint 120 may include to segment portions, i.e., lower portion 1204 and upper portion 1206 forming a rotary joint. Lower portion 1204 moves relative to upper portion 1206 in a rotary fashion. Likewise, upper portion 1206 moves relative to lower portion 1204 in a rotary fashion. In the disclosed example, arm extension 118 is rigidly connected to lower portion 1204 and arm extension 122 is rigidly connected to upper portion 1206 of joint 120. Thus each extension, 118, 122 moves relative to one another in rotary fashion.

Joint 120 may include a locking mechanism 136 for locking articulated arm 128 in a fixed position. For example, locking mechanism 136 may include a handle and a bolt (not shown) centering through joint 120 and in threaded engagement with the handle. Rotating the handle, for example, in a rotational direction, thereby, clamps down on joint 120 as the bolt is threaded into the handle thereby providing enough force to retain the joint and prevent further movement of the same. In the example disclosed, for example, in FIGS. 12 and 13, upon tightening of the aforementioned handle, lower portion 1204 and upper portion 1206 would remain unmovable relative to one another and effectively "lock-down" joint 120 into a fixed or set configuration. Locking mechanism 136 may be configured to lock articulated arm 128, including joints 124, 408, such that articulated arm 128 becomes a completely rigid at a prescribed positioning.

Arm extension 122 connects with joint 120 at a first end and is configured to connect with joint 124 at a second end. Thus, joint 120 forms a joint connection between arm extension 118 and arm extension 122. Joint 124 may include a ball joint wherein arm extension 122 connects with the ball portion of the pivot joint 134. The ball joint permits rotary movement of arm extension 122 in the x-direction, the y-direction, and z-direction (e.g., see FIG. 2). Arm extension 122 is a rigid arm extension that may be comprised of stainless steel, medical grade steel, materials.

An attachment unit 132 serves as a fixation device to connect to an extremity or body portion of a patient. Attachment unit 132 may also be configured to attach to other items used in surgery including, but not limited to, a human body part, a bone screw, or an implant. In the disclosed example shown in FIG. 1, attachment unit 132 is configured as a head clamp designed to couple and attach with the head of a patient. However, it will be readily appreciated by those skilled in the art that attachment unit 132 may be designed to affix to other extremities or portions of a human patient including, for example, arms, legs, knees, angles, neck, wrists, hands, etc. Thus attachment unit 132 may comprise other alternative attachment mechanisms, for example, including attachment configurations for bone screws, spinal clamps, surgical pins, etc., or any other surgical mount suitable for affixing connection unit 126 to a human body part or extremity. The current example depicts a mounting post 130 extending from attachment unit 132 and is configured to connectively attach to joint 124 thereby forming a joint connection between arm extension 122 and attachment unit 132. Thus, joint 124 permits movement of articulated arm 128 and marker carrier unit relative to attachment unit 132 which may be affixed at a prescribed location, for example, on the body of a patient. While a select number of joints have been illustrated in the drawings and described in the specification, more or less joints may be utilized to form the articulated arm of connection unit 126. In addition, other types of features may be utilized in the articulated arm including, for example, a telescopic feature employed in the arm extension for extending or shortening the arm extension along a length of connection unit 126.

As previously mentioned above, arm extension 118 connects to marker carrier body 112 of marker carrier unit 116 at one end and is configured to join with joint 120 at another end. To connect with marker carrier unit 116, arm extension 118 connects with joint 408 thereby forming a joint connection therebetween. Turning to FIG. 4, joint 408 may comprise a ball joint wherein arm extension 118 connects with the ball portion of the pivot joint 412. Thus, the ball joint permits rotary movement of arm extension 118 in the x-direction, the y-direction, and z-direction (e.g., see FIG. 5). FIGS. 12 and 13 illustrate an example of joint 408 represented as a ball and socket joint in which the ball-shaped surface of one rounded member 1200 fits into a cup-like depression of another member 1202. Coupled to joint 408 is an attachment mechanism for coupling arm extension 118 to marker carrier body 112 via joint 408. In the disclosed example, the attachment mechanism comprises an attachment foot 406. Joint 408 permits attachment foot 406 to have a range of motion around an indefinite number of axes having a common center. The disclosed configuration enables attachment foot 406 to move in many planes (almost all directions) as further described below. Earlier described joint 124 may also include the ball joint configuration represented by joint 408 and illustrated, for example, in the exemplary examples of FIGS. 12 and 13.

Arm extension 118 is a rigid arm extension that may be comprised of stainless steel, medical grade steel, materials. As illustrated in FIG. 4, marker element 110 is mounted on mounting post 414 rigidly fixed to marker carrier body 112. Marker carrier body 112 comprises an attachment area 404 for receiving and coupling/mating an attachment mechanism, such as attachment foot 406, in receiving area 402. Receiving area 402 may be formed as a recess or cavity appropriately dimensioned and configured to receive and retain attachment foot 406 therein, as described below. Attachment foot 406 extends from joint 408 via mounting post 410. Marker carrier unit 116 is ultimately retained on connection unit 126 via the receipt and retention of attachment foot 406. Thus, by function of joint 124, connection unit 126 is permitted to rotate and pivot relative to attachment unit 132. By function of joint 408, marker carrier unit 116 is permitted to rotate and pivot relative to connection unit 126.

FIG. 6 illustrates a top view of one example of attachment foot 406 mated in receiving area 402 of marker carrier body 112. When received within receiving area 402, select inner wall portions of marker carrier body 112 are sufficiently designed to contact points of the outer surface of attachment foot 406 to facilitate locating and securing the same therein. For example, in one disclosed example locating contact surfaces 602 are formed to protrude into receiving area 402. Locating contact surfaces 602 act as an alignment mechanism of marker carrier body 112 for positioning onto attachment foot 406, as described below. Appropriately sized receiving areas 612 of attachment foot 406 are configured to receive corresponding locating contact surfaces 602 to form a mated configuration wherein the outer surface 614 of locating contact surfaces 602 generally abuts against the outer surface 616 of corresponding receiving areas 612. Side edge surface 618 of attachment foot 406 also generally abuts corresponding side edges 620 of receiving area 402.

A clamp lever 604 is provided to position and retain attachment foot 406 within receiving area 402. Pin 606 is disposed through clamp lever 604 such that clamp lever 604 pivots about pin 606. (As shown more easily in FIG. 7, pin 606 may be secured within the structure of marker carrier body 112.) When clamp lever 604 is pivoted about pin 606, the outer surface 610 of clamp lever 604 is rotated into contact with outer surface 608 of attachment foot 406 thereby providing a frictional interference fit in a clamped position. A material of attachment foot 406 and/or clamp lever 604 may be designed to withstand a certain amount of deflection to facilitate the frictional fit and retention of clamp lever 604 in the clamped position and thereby secure attachment foot 406 within receiving area 402.

FIG. 7 provides a cross sectional view taken along C - C of FIG. 6. In the exemplary example, a medical drape 700 may be disposed over attachment foot 406 within receiving area 402. As illustrated in the current example, the side circumference 710 of attachment foot 406 may be designed with a generally multi-angular configuration. Thus a top half 712 of the side circumference 710 may angle generally downwardly and away from a top surface 716 of attachment foot 406 to form a top half angled surface 406b. A bottom half 714 of the side circumference 710 may angle generally upwardly and away from a bottom surface 718 of attachment foot 406 to form a bottom half angled surface 406a. Top half angled surface 406b and bottom half angled surface 406a are configured to diverge into a point 702.

Outer surface 614 of protruding locating contact surfaces 602 is designed to mate in complimentary fashion with the design configuration of top half angled surface 406b and the bottom half angled surface 406a. Angled surfaces of outer surface 614 include a top half angled surface 602b and a bottom half angled surface 602a that diverge into point 704. Accordingly, top half angled surface 602b, bottom half angled surface 602a and point 704 of outside surface of contact surface 602 are formed in a complimentary configuration to mate with the angular design of corresponding top half angled surface 406b, bottom half angled surface 406a and point 702, respectively.

Next, the current example of the configuration of outer surface 608 of attachment foot 406 with respect to outer surface 610 of clamp level 604 is described. The side circumference 710 of attachment foot 406 may be designed with a generally multi-angular configuration. Top half 712 of side circumference 710 may angle generally downwardly and away from top surface 716 of attachment foot 406 to form a top half angled surface 406c. Bottom half 714 of side circumference 710 may angle generally upwardly and away from a bottom surface 718 of attachment foot 406 to form a bottom half angled surface 406d. Top half angled surface 406c and bottom half angled surface 406d are configured to diverge at a point 706.

Outer surface 610 of clamp lever 604 is designed to mate in complimentary fashion with the design configuration of top half angled surface 406c and bottom half angled surface 406d. Angled surfaces of outer surface 610 include a top half angled surface 604b and a bottom half angled surface 604a that diverge at point 708. Accordingly, top half angled surface 604b, bottom half angled surface 604a of contact surface 610 and point 708 are formed in a complimentary configuration to mate with the angular design of corresponding top half angled surface 406c, bottom half angled surface 406d and point 706, respectively.

In operation, when clamp lever 604 is pivoted about pin 606 to bring outer surface 610 into contact with outer surface 608 of attachment foot 406, top half angled surface 406b, bottom half angled surface 406a and point 702 of attachment foot 406 mate with top half angled surface 602b, bottom half angled surface 602a and point 704 of outside surface 614 of locating contact surface 602, respectively. In this manner, locating contact surface 602 provides an alignment mechanism of marker carrier body 112 of the disclosed invention. This ensures that any marker carrier body 112 employing the designed receiving area 402 and the locating contact surfaces 602 will always be in the same position, location and/or orientation when mounted on the disclosed attachment foot 406 having corresponding complimentary receiving areas 612 after articulated arm 128 is set into a final position. Likewise, top half angled surface 604b, bottom half angled surface 604a and point 708 of contact surface 610 mate with the angular design of corresponding top half angled surface 406c, bottom half angled surface 406d and point 706 of attachment foot 406, respectively.

Turning to FIG. 8, another example of attachment foot 406 mated in receiving area 402 of an exemplary marker carrier body 112 is shown. Attachment foot 406 is mated in receiving area 402 of marker carrier body 112. When received within receiving area 402, select inner wall portions of marker carrier body 112 are sufficiently designed to contact points of the outer surface of attachment foot 406 to facilitate locating and securing the same therein. For example, general angular side contact surfaces 802 are formed at a complimentary angle to side angular contact surfaces 810 of attachment foot 406. Angular side contact surfaces 802 are connected via a forward front surface 804. Forward front surface 804 corresponds to a complimentary forward surface 806 of attachment foot 406. Angular side contact surfaces 802 and forward front surface 804 act as an alignment mechanism of marker carrier unit 112. Thus, when clamp lever 604 is rotated about pin 606, outer surface 610 of clamp lever 604 is urged against rearward surface 812 of attachment foot 406. This motion urges forward surface 806 of attachment foot 406 into contact with forward front surface 804. Additionally, side angular contact surfaces 810 of attachment foot 406 abut angular side contact surfaces 802 of marker carrier body 112.

In the cross sectional view of FIG. 9 a medical drape 700 is disposed over attachment foot 406 within receiving area 402. As illustrated in the current example, the side circumference 908 of attachment foot 406 may be designed with a generally angular configuration. Thus, at a location disposed near forward surface 806, a top half 912 of side circumference 908 may angle generally downwardly and inwardly from a top surface 914 of attachment foot 406 to form a top half angled surface 902b near forward surface 806. A bottom half 916 of the side circumference 908 may angle generally upwardly and inwardly from a bottom surface 918 of attachment foot 406 to form a bottom half angled surface 902a near forward surface 806. Top half angled surface 902b and bottom half angled surface 902a near forward surface 806 are configured to diverge into a point 910.

Forward front surface 804 acts as an abutment surface and is designed to mate in complimentary fashion with the configuration of top half angled surface 902b and bottom half angled surface 902a of attachment foot 406. Angled surfaces of forward front surface 804 include a top half angled surface 802b and a bottom half angled surface 802a that diverge into point 904. Accordingly, top half angled surface 802b, bottom half angled surface 802a and point 904 of forward front surface 804 are formed in a complimentary configuration to mate with the angular design of corresponding top half angled surface 902b, bottom half angled surface 902a and point 910, respectively.

Next, the current example of the configuration of rearward surface 812 of attachment foot 406 with respect to outer surface 610 of clamp level 604 is described. The side circumference 908 of attachment foot 406 may be designed with a generally angular configuration. Top half 912 of side circumference 908 may angle generally downwardly and away from top surface 914 of attachment foot 406 to form a top half angled surface 902c. Bottom half 916 of side circumference 908 may angle generally upwardly and away from a bottom surface 918 of attachment foot 406 to form a bottom half angled surface 902d. Top half angled surface 902c and bottom half angled surface 902d are configured to diverge at a point 906.

Outer surface 610 of clamp lever 604 is designed to mate in complimentary fashion with the design configuration of top half angled surface 902c and bottom half angled surface 902d. Angled surfaces of outer surface 610 include a top half angled surface 604b and a bottom half angled surface 604a that diverge at point 920. Accordingly, top half angled surface 604b, bottom half angled surface 604a and point 920 of contact surface 610 are formed in a complimentary configuration to mate with the angular design of corresponding top half angled surface 902c, bottom half angled surface 902d and point 906, respectively.

In operation, when clamp lever 604 is pivoted about pin 606 to bring outer surface 610 into contact with rearward surface 812 of attachment foot 406, top half angled surface 902b, bottom half angled surface 902a and point 910 of attachment foot 406 mate with top half angled surface 802b, bottom half angled surface 802a and point 904 of forward front surface 804, respectively. In this manner, forward front contact surface 804 and in combination with side contact surfaces 802 provide an alignment mechanism of marker carrier body 112. This ensures that any marker carrier body 112 employing the designed receiving area 402 and front contact surface 804 in combination with side contact surfaces 802 will always be in the same position, location and/or orientation when mounted on the disclosed attachment foot 406 having complimentary forward surface 806 and angular contact surfaces 810 after articulated arm 128 is set into a final position. Likewise, top half angled surface 604b, bottom half angled surface 604a and point 920 of contact surface 610 mate with the angular design of corresponding top half angled surface 902c, bottom half angled surface 902d and point 906 of attachment foot 406, respectively.

FIGS. 10 and 11 illustrate alternate examples of the attachment foot 406 and clamp lever 604 design. Turning to FIG. 10, a cross-sectional view of attachment foot 406 is disposed within receiving area 402. A medical drape 700 may be disposed over attachment foot 406 within receiving area 402. The side circumference 710 of attachment foot 406 may be designed with a generally multi-angular configuration. Thus a top half 712 of the side circumference 710 may angle generally downwardly and away from a top surface 716 of attachment foot 406 to form a top half angled surface 406b. A bottom half 714 of the side circumference 710 may angle generally upwardly and away from a bottom surface 718 of attachment foot 406 to form a bottom half angled surface 406a. Top half angled surface 406b and bottom half angled surface 406a are configured to diverge into a point 702.

The side wall surface 1012 of receiving area 402 is configured to mate with the surface of side circumference 710. An angular side surface 1002 of side wall surface 1012 is designed at a complimentary angle to mate in complimentary fashion with the bottom half angled surface 406a. Thus, surfaces of side wall surface 1012 include a wall 1006 extending downwardly and generally perpendicular from a top surface 1008 of receiving area 402. Side wall surface 1012 also includes angular side surface 1002 angled downwardly and inwardly from a point 1010 extending from a bottom of wall 1006. Accordingly, angular side surface 1002 and point 1010 of side wall surface 1012 are formed in a complimentary configuration to mate with the angular design of corresponding bottom half angled surface 406a and point 702, respectively.

Next, the current example of the configuration of outer surface 608 of attachment foot 406 with respect to outer surface 610 of clamp level 604 is described. The side circumference 710 of attachment foot 406 may be designed with a generally multi-angular configuration. Top half 712 of side circumference 710 may angle generally downwardly and away from top surface 716 of attachment foot 406 to form a top half angled surface 406c. Bottom half 714 of side circumference 710 may angle generally upwardly and away from a bottom surface 718 of attachment foot 406 to form a bottom half angled surface 406d. Top half angled surface 406c and bottom half angled surface 406d are configured to diverge at a point 706.

Outer surface 610 of clamp lever 604 is designed to mate in complimentary fashion with the design configuration of bottom half angled surface 406d. Outer surface 610 includes a wall 1014 extending downwardly and generally perpendicular from a top surface 1012 of clamp lever 604. Outer surface 610 also includes angular side surface 1000 angled downwardly and inwardly from a point 1004 extending from a bottom of wall 1014. Accordingly, angular side surface 1000 and point 1004 of outer surface 610 are formed in a complimentary configuration to mate with the angular design of corresponding bottom half angled surface 406d and point 706, respectively.

In operation, when clamp lever 604 is pivoted about pin 606 to bring outer surface 610 into contact with outer surface 608 of attachment foot 406, bottom half angled surface 406a and point 702 of attachment foot 406 align with angular side surface 1002 and point 1010 of side wall surface 1012, respectively. Likewise, top half angled surface 604b, bottom half angled surface 604a and point 708 of contact surface 610 mate with the angular design of corresponding top half angled surface 406c, bottom half angled surface 406d and point 706 of attachment foot 406, respectively. In a final assembly, top surface 716 of attachment foot 406 may be abutted against top surface 1008 of receiving area 402 to secure attachment foot 406 within receiving area 402. In this manner, this ensures that any marker carrier body 112 employing the designed receiving area 402 of FIG. 10 will always be in the same position, location and/or orientation when mounted on the disclosed attachment foot 406 of FIG. 10 after articulated arm 128 is set into a final position.

Turning to an alternate example depicted in FIG. 11, a cross-sectional view illustrates attachment foot 406 disposed within receiving area 402. A medical drape 700 is disposed over attachment foot 406 within receiving area 402. The side circumference 710 of attachment foot 406 may be designed with a generally angular configuration. A side profile of attachment foot 406 generally represents a trapezoidal shape wherein a top surface 1118 is slightly longer than a bottom surface 1120 of attachment foot 406. As shown, top surface 1118 is connected to bottom surface 1120 via a generally downwardly and inwardly angular wall surface 1102 disposed near angular side wall 1104 of receiving area 402. Point 1114 is formed at the joint wherein angular wall surface 1102 extends from top surface 1118. Angular side wall 1104 extends from a top surface 1112 of receiving area 402. Angular side wall 1104 may extend downwardly and inwardly at an angle complimentary to the angle formed by angular wall surface 1102. Point 1116 is formed at the joint wherein angular side wall 1104 extends from top surface 1112 of receiving area 402.

Likewise, top surface 1118 is connected to bottom surface 1120 via a generally downwardly and inwardly angular wall surface 1106 disposed near outer surface 610 of clamp lever 604. Outer surface 610 may form an angular surface 1108 generally complimentary to the angle formed by angular wall surface 1106. In operation, when clamp lever 604 is pivoted about pin 606 to bring angular surface 1108 into contact with angular wall surface 1106 of attachment foot 406, angular wall surface 1102 is brought into alignment with angular side wall 1104 and point 1114 meets with point 1116 in the aligned configuration. Additionally, angular surface 1108 is aligned with angular wall surface 1106, and top surface 1118 abuts top surface 1112 to retain attachment foot within fixed position of receiving area 402. In this manner, this ensures that any marker carrier body 112 employing the designed receiving area 402 of FIG. 11 will always be in the same position, location and/or orientation when mounted on the disclosed attachment foot 406 of FIG. 11 after articulated arm 128 is set into a final position.

Given the improved features provided by examples of the disclosed referencing device 100, a method for preparing an image-guided, surgical navigation system is outlined herein. Turning to FIG. 14, a technique 1400 for preparing and utilizing an image-guided surgical navigation according to disclosed examples is depicted. Step 1402 requires fixing attachment unit 132 of referencing device 100 onto a patient. Step 1404 includes installing marker carrier body 112 onto connection unit 126. This may include adjusting components of connection unit 126 into a preferred position to orient marker carrier body 112 into a prescribed location, position and/or orientation. Adjustment of connection unit 126 may include manipulating arm extensions 118, 122 and joints 120, 124, 408, as necessary. Once an acceptable position is achieved, for example, an acceptable position of articulated arm 128 and orientation of marker carrier body 112, the articulated arm may be locked into a final position via locking mechanism 136 to secure the orientation of referencing device 100. Step 1406 includes registering a position and orientation of referencing device 100. This may include specifically registering a location, position and/or orientation of marker carrier unit 116. Having connection unit 126 locked into position, claim lever 604 may act as a detachment mechanism for releasing marker carrier body 112 from attachment foot 406. Accordingly, step 1408 includes detaching marker carrier body 112 from connection unit 126. Step 1410 provides draping the patient and connection unit 126 of referencing device 100. Disclosed examples provide that connection unit 126 includes articulated arm 126 including joint 408 and attachment foot 406. As illustrated in, at least, FIGS. 7, 9, 10, and 11, medical drape 700 is disposed over attachment foot 406 (and in a final assembly within receiving area 402). Step 1412 includes attaching a sterile marker carrier body 112 to connection unit 126. The sterile marker carrier body 112 may be the previous marker carrier body 112 which has since been sterilized, or it may be another sterile marker carrier body 112. A design of the disclosed marker carrier body 112 provides a uniquely configured receiving area that automatically locates, positions and orients marker carrier body 112 on a mountable attachment foot 406 in a complimentary mated fashion. The disclosed design consistently orients marker carrier body 112 to a repeatable prescribed position mounted on a complimentary configured attachment foot 406. The mounting and securing of marker carrier body 112 includes a feature of positioning and affixing medical draping in a secure and consistent manner. Once a sterile marker carrier body 112 is fixed mounted to connection unit 126, step 1414 includes starting a navigation procedure via an image-guided surgery (IGS).

Disclosed examples may provide certain indicia and/or colors on components of the disclosed invention such as, but not limited to, marker carrier unit 116, marker carrier body 112, and attachment foot 406. In one example, the aforementioned indicia and/or colors may correspond to a specific use or application associated with said indicia and/or colors. Such specific uses or applications associated with said indicia and/or colors may be employed, for example, in specific prescribed distinct surgical procedures or in certain environments or medical situations. These may include, but not limited to, for example, use in neuro and ENT surgery, spinal applications, soft/sensitive tissue applications and/or applying force applications. Furthermore, it should be appreciated that all examples in the present disclosure, while illustrating many examples of the present invention, are provided as non-limiting examples and are, therefore, not to be taken as limiting the various aspects so illustrated.

## Claims

1. A device for a surgical navigation system, comprising:
a connection unit (126), wherein the connection unit comprises an articulated arm (128), and wherein the articulated arm comprises:
a first arm extension (118) having a first end and a second end;
a second arm extension (122) having a first end and a second end;
a first joint connection (120) between the first end of the first arm extension (118) and the first end of the second arm extension (122), thereby connecting the first arm extension to the second arm extension;
a second joint connection (412) disposed at the second end of the first arm extension (118); and
a third joint connection (124) disposed at the second end of the second arm extension (122),
wherein the first joint connection (120) is a rotary joint, the second joint connection (412) is a ball joint, and the third joint connection (124) is a ball joint);
a marker carrier unit (116) removably attached to the connection unit (126);
an attachment unit (132) connected to the connection unit (126) for fixing the device to a body part of a patient,
wherein the marker carrier unit (116) comprises an attachment area (404) for removably attaching the marker carrier unit to the connection unit (126), and the connection unit further comprising an attachment mechanism (406) configured at the second end of the first arm extension and configured to couple to the attachment area of the marker carrier unit,
wherein the attachment mechanism (406) comprises an attachment foot (406), and the attachment foot is connected to the second joint connection (412)
wherein the second joint connection (412) permits the marker carrier unit (116) in mated connection with the attachment foot (406) to be rotated and pivoted relative to the connection unit (126),
wherein the marker carrier unit (116) comprises an alignment mechanism (602) in connection with the attachment foot (406) to position and orient the marker carrier unit (116) relative to the connection unit (126),
wherein the attachment foot (406) comprises receiving areas (612) corresponding to the alignment mechanism (602) of the marker carrier unit (116), wherein the alignment mechanism and receiving areas cooperate to align the marker carrier unit in connection with the attachment foot to position and orient the marker carrier unit relative to the connection unit, and
further comprising a clamp lever (604) secured to a surface of the marker carrier unit (116) and disposed near a position of the attachment area (404),
wherein the clamp lever pivots to engage and lock the attachment foot within the attachment area of the marker carrier unit thereby providing a secure connection to position and orient the marker carrier unit.

2. The device of claim 1, wherein a side circumference (710) of the attachment foot (406) matches a complementary profile of the alignment mechanism (602) and a surface (610) of the clamp lever (604) engaged with the attachment foot.

## Patentansprüche

1. Vorrichtung für ein chirurgisches Navigationssystem, umfassend:
eine Verbindungseinheit (126), wobei die Verbindungseinheit einen Knickarm (128) umfasst, und wobei der Knickarm Folgendes umfasst:
einen ersten Armfortsatz (118) mit einem ersten Ende und einem zweiten Ende;
einen zweiten Armfortsatz (122) mit einem ersten Ende und einem zweiten Ende;
eine erste Gelenkverbindung (120) zwischen dem ersten Ende des ersten Armfortsatzes (118) und dem ersten Ende des zweiten Armfortsatzes (122), wodurch der erste Armfortsatz mit dem zweiten Armfortsatz verbunden ist;
eine zweite Gelenkverbindung (412), die an dem zweiten Ende des ersten Armfortsatzes (118) angeordnet ist; und
eine dritte Gelenkverbindung (124), die an dem zweiten Ende des zweiten Armfortsatzes (122) angeordnet ist,
wobei die erste Gelenkverbindung (120) ein Drehgelenk ist, die zweite Gelenkverbindung (412) ein Kugelgelenk ist und die dritte Gelenkverbindung (124) ein Kugelgelenk ist;
eine Markierungsträgereinheit (116), die abnehmbar an der Verbindungseinheit (126) angebracht ist;
eine Anbringungseinheit (132), die mit der Verbindungseinheit (126) verbunden ist, zum Fixieren der Vorrichtung an einem Körperteil eines Patienten,
wobei die Markierungsträgereinheit (116) einen Anbringungsbereich (404) zum abnehmbaren Anbringen der Markierungsträgereinheit an der Verbindungseinheit (126) umfasst und die Verbindungseinheit weiterhin einen Anbringungsmechanismus (406) umfasst, der an dem zweiten Ende des ersten Armfortsatzes eingerichtet ist und dazu eingerichtet ist, sich an den Anbringungsbereich der Markierungsträgereinheit zu koppeln,
wobei der Anbringungsmechanismus (406) einen Anbringungsfuß (406) umfasst und der Anbringungsfuß mit der zweiten Gelenkverbindung (412) verbunden ist,
wobei die zweite Gelenkverbindung (412) ermöglicht, dass die Markierungsträgereinheit (116) in einer Steckverbindung mit dem Anbringungsfuß (406) in Bezug auf die Verbindungseinheit (126) gedreht und geschwenkt werden kann,
wobei die Markierungsträgereinheit (116) einen Abgleichmechanismus (602) in Verbindung mit dem Anbringungsfuß (406) umfasst, um die Markierungsträgereinheit (116) in Bezug auf die Verbindungseinheit (126) zu positionieren und auszurichten,
wobei der Anbringungsfuß (406) Aufnahmebereiche (612) umfasst, die dem Abgleichmechanismus (602) der Markierungsträgereinheit (116) entsprechen, wobei der Abgleichmechanismus und die Aufnahmebereiche zusammenwirken, um die Markierungsträgereinheit in Verbindung mit dem Anbringungsfuß abzugleichen, um die Markierungsträgereinheit in Bezug auf die Verbindungseinheit zu positionieren und auszurichten, und
weiterhin umfassend einen Klemmhebel (604), der an einer Oberfläche der Markierungsträgereinheit (116) gesichert ist und nahe einer Position des Anbringungsbereichs (404) angeordnet ist,
wobei der Klemmhebel schwenkt, um den Anbringungsfuß innerhalb des Anbringungsbereichs der Markierungsträgereinheit in Eingriff zu bringen und zu arretieren, wodurch eine sichere Verbindung bereitgestellt wird, um die Markierungsträgereinheit zu positionieren und auszurichten.

2. Vorrichtung nach Anspruch 1, wobei ein Seitenumfang (710) des Anbringungsfußes (406) mit einem komplementären Profil des Abgleichmechanismus (602) und einer Oberfläche (610) des Klemmhebels (604), der mit dem Anbringungsfuß in Eingriff steht, übereinstimmt.

## Revendications

1. Dispositif pour un système de navigation chirurgicale, comprenant :
une unité de raccordement (126), l'unité de raccordement comprenant un bras articulé (128) et le bras articulé comprenant :
une première extension de bras (118) ayant une première extrémité et une deuxième extrémité ;
une deuxième extension de bras (122) ayant une première extrémité et une deuxième extrémité ;
une première liaison articulée (120) entre la première extrémité de la première extension de bras (118) et la première extrémité de la deuxième extension de bras (122) afin de raccorder ainsi la première extension de bras à la deuxième extension de bras ;
une deuxième liaison articulée (412) disposée à la deuxième extrémité de la première extension de bras (118) ; et
une troisième liaison articulée (124) disposée à la deuxième extrémité de la deuxième extension de bras (122),
dans lequel la première liaison articulée (120) est un joint rotatif, la deuxième liaison articulée (412) est un joint à rotule et la troisième liaison articulée (124) est un joint à rotule ;
une unité support de marqueur (116) fixée de manière amovible à l'unité de raccordement (126) ;
une unité de fixation (132) raccordée à l'unité de raccordement (126) pour fixer le dispositif à une partie du corps d'un patient,
dans lequel l'unité support de marqueur (116) comprend une zone de fixation (404) pour fixer de manière amovible l'unité support de marqueur à l'unité de raccordement (126), et l'unité de raccordement comprend en outre un mécanisme de fixation (406) configuré à la deuxième extrémité de la première extension de bras et configuré pour se coupler à la zone de fixation de l'unité support de marqueur,
dans lequel le mécanisme de fixation (406) comprend un pied de fixation (406) et le pied de fixation est raccordé à la deuxième liaison articulée (412),
dans lequel la deuxième liaison articulée (412) permet à l'unité support de marqueur (116) accouplée au pied de fixation (406) de tourner et de pivoter par rapport à l'unité de raccordement (126),
dans lequel l'unité support de marqueur (116) comprend un mécanisme d'alignement (602) raccordé au pied de fixation (406) pour positionner et orienter l'unité support de marqueur (116) par rapport à l'unité de raccordement (126),
dans lequel le pied de fixation (406) comprend des zones de réception (612) correspondant au mécanisme d'alignement (602) de l'unité support de marqueur (116), dans lequel le mécanisme d'alignement et les zones de réception coopèrent pour aligner l'unité support de marqueur raccordée au pied de fixation afin de positionner et orienter l'unité support de marqueur par rapport à l'unité de raccordement, et
comprenant en outre un levier de serrage (604) fixé à une surface de l'unité support de marqueur (116) et disposé proche d'une position de la zone de fixation (404),
dans lequel le levier de serrage pivote pour venir en prise avec et bloquer le pied de fixation à l'intérieur de la zone de fixation de l'unité support de marqueur, réalisant ainsi un raccordement sûr pour positionner et orienter l'unité support de marqueur.

2. Dispositif selon la revendication 1, dans lequel une circonférence latérale (710) du pied de fixation (406) correspond à un profil complémentaire du mécanisme d'alignement (602) et à une surface (610) du levier de serrage (604) en prise avec le pied de fixation.
